# EUROPEAN PATENT APPLICATION

(11) **EP 4 438 085 A1**
(43) Date of publication of application: **02.10.2024**
(21) Application number: 23165348.6
(22) Date of filing: 30.03.2023
(51) Int. Cl.: A61M 5/32

(54) **A NEEDLE SHIELD REMOVER**

(71) Applicant: Ypsomed AG, 3401 Burgdorf (CH)
(72) Inventor: Scheurer, Simon, 3006 Bern (CH); Hostettler, Patrick, 3415 Hasle (CH); Allenspach, Marcel, 3400 Burgdorf (CH); Klötzli, Urs, 3414 Oberburg (CH); Zumstein, Dominik, 3014 Bern (CH)
(74) Representative: Meier Obertüfer, Jürg

(57) **Abstract**

A needle shield remover (1) for a medicament delivery device comprising a needle shield (19). The needle shield remover comprises a cap (2) with a side wall (3) providing a bore (4), the proximal section (5) of the side wall (3) is configured for attachment to the medicament delivery device. An elastic sleeve (6) positioned in the bore (4) comprising a holding section (7) for holding the needle shield. The needle shield remover comprises a distal end wall (9) connecting the elastic sleeve (6) to the cap (2). A passage (10) is provided in the distal end wall (9) connecting the inner space of the elastic sleeve (6) to the exterior, the passage (10) being configured for receiving an expansion tool (16) for expanding the elastic sleeve to fit around the needle shield.

## Description

### TECHNICAL FIELD

The current invention relates to a needle shield remover that is configured to remove a needle shield from a medicament delivery device and to a method for applying a needle shield remover to a medicament delivery device.

### BACKGROUND OF THE INVENTION

Administering liquid medication for subcutaneous or intravenous delivery may be done using a syringe. The hollow needle of the syringe may be covered by a needle shield to prevent contamination of the medicament present in the syringe. The tightness of the connection between the needle shield and the syringe is tested using so-called container closure integrity (CCI) testing where the ingress of a contaminant into the syringe is tested. Using a syringe for the injection process may present a number of risks and challenges to the user or the health care professional as a manual force needs to be applied to a plunger in the syringe during an injection while holding the syringe and after the injection the needle may be exposed increasing the risk of needle sticking.

Medicament delivery devices have been developed for the delivery of a liquid medicament from a syringe or a reservoir such as a cartridge. The delivery devices may be auto injection devices such as autoinjectors or patch injectors and those have been developed to facilitate the injection process and reduce the risks for the user or health care professional using these delivery devices.

The medicament delivery devices are prefilled with a syringe including the needle shield and the medicament delivery device may require a needle shield remover that the user or health care professional removes from the device just prior to use thereby also removing the needle shield from the syringe. The needle shield remover needs to be assembled with the auto injection device during manufacturing for obtaining the prefilled device. The assembly of the needle shield remover with the needle shield, which is already present on the syringe, should maintain the integrity of the connection between the syringe and the needle shield ensuring CCI for the syringe in the assembled and prefilled medicament delivery device.

EP2023983B1 discloses a needle shield remover comprising a cap constructed from a plastic material with a metal insert including barbed hooks. A medicament delivery device including a prefilled syringe closed by a flexible needle needs to be assembled with the cap. The syringe with the needle shield is inserted into the cap with the metal insert thereby deflecting the barbed hooks and anchoring the flexible needle shield to the cap via the insert. During assembly, axial forces impact the needle shield and this may affect the container closure integrity of the prefilled syringe in the medicament delivery device after assembly with the needle shield remover.

EP1755706B2 discloses a needle shield remover including a cap made from a plastic material and a metal insert shaped as a castellated washer for engaging a rigid needle shield positioned at the end of a prefilled syringe of an autoinjector. During assembly the washer engages the rigid needle shield thereby applying axial forces to the needle shield that may affect the CCI.

US 10518042BB discloses a removable needle cap for an autoinjector, the needle cap being constructed from one material. The body of the needle cap is based on a heat shrinkable tube or constructed from an elastic material or constructed from an elastic fabric that is shrunk around the needle shield when pulling (removing) the needle cap. Low, or no elastic radial directed shrinking forces are applied on the needle shield during assembly with the needle cap.

US11318259BB discloses a needle shield remover having a generally tubular body with a longitudinal extension and a grip element composed of a braided sleeve having an opening of such a diameter that a needle shield may be introduced during assembly, and where the diameter of the braided sleeve is reduced such that a friction fit is created between the braided sleeve and the needle shield when said needle shield remover is pulled and contracted for removing the needle shield.

It is an object of the present invention to provide an improved and reliable needle shield remover overcoming the drawbacks of the prior art. The needle shield remover is provided as one part without the need of metal inserts and is configured to apply radial forces onto the needle shield during assembly. Furthermore a manufacturing method applying the needle shield remover to a medicament delivery is disclosed. These objectives are solved by the independent claims and specific variants are disclosed in the dependent claims.

### DEFINITIONS

The term "medicament" or "medication" includes any flowable medical formulation suitable for controlled administration through a means such as, for example, a cannula or a hollow needle and includes a liquid, a solution, a gel or a fine suspension containing one or more medical active ingredients. A medicament can be a composition including a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the hollow needle configured to penetrate the skin of the patient. For a medicament delivery device such as an injection pen or auto injector this may be the injection needle and the end of the pen or auto injector holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

In the claims, the word "comprising" does not exclude other elements or steps, and the indefinite article "a" or "an" does not exclude a plurality. For example "an overlap area" does not exclude the fact that there may be two overlap areas that functionally or structurally fulfill the purpose of "an overlap area". The mere fact that certain elements or steps are recited in distinct claims shall not preclude the existence of further meaningful combinations of these elements or steps.

### GENERAL DESCRIPTION OF THE INVENTION

The current disclosure relates to a needle shield remover for a medicament delivery device. The needle shield remover intends to remove a needle shield. The medicament delivery device includes a syringe with an attached needle to the distal end and the needle, or at least a part of the needle, is covered by the needle shield. The needle shield remover includes a cap having a side wall, the side wall provides a bore and a proximal section of the side wall is configured for attachment to the medicament delivery device. The needle shield remover further includes an elastic sleeve positioned within the bore and the elastic sleeve includes a holding section for receiving and/or holding the needle shield. The elastic sleeve or at least the holding section of the elastic sleeve has an inner dimension, for example inner diameter that is smaller than the maximum outer dimension, for example the maximum outer diameter of the needle shield. The elastic sleeve includes a wall that is constructed from an elastic material. The needle shield remover includes a distal end wall connecting the elastic sleeve to the cap and a passage is provided in the distal end wall connecting the inner space of the elastic sleeve and/or the inner space of the holding section to the exterior. The passage in the distal end wall is configured for receiving an expansion tool for expanding the elastic sleeve or at least the holding section of the elastic sleeve to an expanded state, preferably a radially expanded state. The inner dimension of the passage in the end wall is greater than the outer diameter of the needle shield such that the expansion tool may be inserted into the passage thereby expanding the elastic sleeve and/or the holding section of the elastic sleeve from the non-expanded state to a radially expanded state. The inner dimension of the passage in the end wall is greater than the outer dimension of the needle shield such that there is a clearance between the passage and the needle shield that is available for insertion of the expansion tool.

The medicament delivery device may be an injection device such as an auto injector or a patch injector or bolus injector. The medicament delivery device may be an infusion device. The syringe may be a prefilled syringe with a stacked needle or the syringe may have a hub for releasable connection of a needle. The needle may be a hollow needle or a cannula. The distal end of the needle may be covered by the needle shield protecting the needle from contamination. The needle shield may fully cover the needle or may cover a part of the needle including the distal tip of the needle. The needle shield may cover a distal end of the syringe that is adapted to hold the needle. The needle shield may be constructed from an elastomeric or flexible material, a so-called flexible needle shield, or the needle shield includes a flexible component covering the needle and an outer component that is less flexible compared to the inner part. The inner and outer component together provide for the so-called rigid needle shield.

The proximal end or a proximal section of the side wall of the cap is configured for attachment to the medicament delivery device or to a housing or housing part of the medicament delivery device. The housing for the medicament delivery device encloses a delivery mechanism for medicament delivery, for example a spring driven delivery mechanism. The engagement between the housing and the cap may be a releasable friction-fit or a releasable from-fit engagement, for example using elastic arms, hooks or latching elements. As an alternative option the engagement is a screw type of engagement.

The elastic sleeve has a proximal end and a distal end and defines a longitudinal axis for the needle shield remover. The proximal end of the elastic sleeve may be oriented towards the proximal direction and the distal end of the sleeve may be attached or attachable to the cap. The elastic sleeve may be coaxially arranged within the cap. The proximal end of the elastic sleeve may be configured to receive the needle shield.

The holding section may be a part of the elastic sleeve and may be located close to the proximal end of the sleeve, and the elastic sleeve or the holding section may have an opening configured to receive the needle shield. The opening may be at the proximal end of the elastic sleeve. The holding section may have an inner dimension in a relaxed, non-deformed state smaller than the maximum radial outer dimension of the needle shield. The holding section of the elastic sleeve may be deformed into a deformed state such that the inner dimension is greater than the maximum outer radial dimension of the needle shield.

The holding section is configured to hold the outer surface of the needle shield by an interference fit and/or a friction fit engagement. Preferably the inner surface of the holding section of the elastic sleeve is configured to hold the outer surface of the needle shield.

The holding section of the elastic sleeve may be expandable from a non-deformed or relaxed state with an inner dimension smaller compared to the maximum outer dimension of the needle shield to an expanded state with an inner dimension greater than the maximum outer dimension of the needle shield. After expansion of the holding section, the needle shield may fit into the holding section and after removing the expansion tool, the holding section may relax to the non-deformed state or to a partially deformed state while holding the needle shield in the friction fit or form fit engagement. Moving from the deformed state to a non-deformed or relaxed state is a radial transition such that only radial forces act on the needle shield.

The elastic sleeve and/or the holding section may include a continuous or non-continuous wall. The wall may be reversibly elastically deformable. A non-continuous wall may be constructed from a woven structure including openings providing a braided structure. A continuous wall may be provided with a closed wall as a monolithic component constructed from an elastic material.

The distal end of the elastic sleeve may be connected or connectable to an inner surface of the end wall. The inner surface of the end wall faces in the proximal direction whereas the outer surface of the end wall faces the distal direction.

The passage in the distal end wall may be an opening or aperture connecting the inner space of the elastic sleeve and the holding section to the exterior. The opening at the distal end of the elastic sleeve is preferably aligned with and connected to the passage in the end wall. The passage may be configured to receive an expansion tool, for expanding the elastic sleeve or at least a part of the elastic sleeve, preferably the holding section of the elastic sleeve. The expansion tool is configured to fit within the passage and is configured to expand the elastic sleeve without expanding the passage in the end wall.

The inner dimension of the passage, preferably the inner dimension at the location of end wall, may accommodate the needle shield, or the inner dimension of the passage, for example the inner diameter, is greater than the maximum outer dimension, for example the maximum outer diameter, of the needle shield.

The inner dimension of the passage, preferably at the location of the end wall, may accommodate the expansion tool, thereby allowing for insertion of the expansion tool into the elastic sleeve via the passage towards the holding section. The inner dimension of the passage, for example the inner diameter, may be greater than the maximum outer dimension of the expansion tool.

The inner dimension of the expansion tool, for example the inner diameter may be greater than the maximum outer dimension, for example the maximum outer diameter, of the needle shield.

The expansion tool may be inserted into the elastic sleeve via the passage in the end wall and during insertion, the expansion tool may expand the elastic sleeve and/or the holding section of the elastic sleeve from the non-expanded to an expanded state. The needle shield may fit into the expansion tool as the inner dimension of the expansion tool is greater than the maximum outer dimension of the needle shield. After removing the expansion tool, the holding section at least partially relaxes and envelopes at least a part of the outer surface of the needle shield.

The expansion tool may have fixed dimensions, for example a fixed inner and outer diameter of a tube or sleeve, or the expansion tool may be provided with a non-expanded state and an expanded state. The expanded state is preferably a radially expanded state.

The radial dimensions are defined in a direction perpendicular to the axis of the elastic sleeve.

In the current disclosure a one part needle shield remover not requiring additional and separate parts like the metal parts of the prior art is presented. The metal holding parts of the prior art may expose radial and axial forces on the needle shield during assembly whereas the holding section in the current disclosure is configured to expose the needle shield to radial forces only. This may improve the container closure integrity for the needle shield or the syringe holding the needle shield when the needle shield remover is applied to the needle shield.

The passage in the end wall allows for introduction of the expansion tool and this facilitates the mounting of the needle shield remover onto the needle shield. Furthermore, the holding section allows for the friction fit/form fit engagement with the outer surface of the needle shield such that the needle shield can easily be removed together with the needle shield remover from the medicament delivery device.

The needle shield remover may be produced by multi-component injection molding, for example two-component injection molding of two different thermoplastic polymers for the cap and for the elastic sleeve whereby an elastomer is selected for the elastic sleeve. The cap may be injection molded first followed by molding the elastic sleeve or, vice versa, the elastic sleeve is injection molded first followed by injection molding of the cap. The needle shield remover may be produced using injection molding of three or four different polymers. Multi-component injection molding may provide for easy manufacturing using less separate parts such as the metal parts of the prior art.

The elastic sleeve may be provided as a cylindrical sleeve comprising a flange that depends radially, or extends radially outward from the distal end of the sleeve. As an alternative, the elastic sleeve has a rectangular or triangular shaped cross section. The wall of the elastic sleeve may include rippled structures or zig-zag structures.

The side wall of the cap may be provided as a cylinder and the end wall may depend radially inward, or may extend radially inward from the side wall. The cross section for the side wall of the cap may be circular for a cylindrical shaped wall of the cap or may be triangular or rectangular shaped.

The flange of the elastic sleeve and the end wall are connected to each other in an overlap area, the overlap area being part of the end wall. The flange of the elastic sleeve that is radially outward directed and the end wall of the cap that is radially inward directed may be connected to each other in an overlap area. In the overlap area an outer surface of the flange of the sleeve engages an outer surface of the end wall of the cap. The overlap area or the surfaces of the flange of the elastic sleeve and the surface of flange of the cap may be structured to improve the mechanical interlock between the two flanges and provide for efficient load transfer from the cap to the elastic sleeve. The load may be transferred effectively to the holding section holding a needle shield. The overlap area may be located adjacent to, or surround the passage in the end wall.

Injection molding of the needle shield remover is preferably executed with no or almost no mold release agents as this may affect the engagement, for example reduce the adhesion, between the polymers in the overlap area.

The elastic sleeve may include an entrance section configured for receiving the distal end of the expansion tool, the entrance section may be positioned or located between the holding section and the flange of the elastic sleeve. The entrance section may have a conical shape starting with a diameter that equals the diameter of the passage in the end wall and ending with an inner diameter equal to the inner diameter of the holding section. The entrance section may have a trumpet shape.

The wall of the elastic sleeve may have a fixed wall thickness or a variable wall thickness. The wall thickness for the entrance section may be greater than the wall thickness of the holding section. The wall thickness of the flange may be smaller than the wall thickness of the holding section. An increased wall thickness for the entrance section may increase the mechanical strength of the elastic sleeve and may be beneficial for entering the expansion tool and preventing damage to the elastic sleeve during inserting of the expansion tool towards the holding section. Further expansion aids may be provided such as applying pressurized gas between the outside surface of the expansion tool and the inner surface of the elastic sleeve providing for an air cushion. Optionally, the surface of the expansion tool is coated with a friction reducing coating such a fluor-based coating such as a PTFE coating.

In an embodiment, the holding section of the elastic sleeve, more preferably the inner surface of the holding section configured for engaging the needle shield, is roughened or textured. This may improve the friction fit between the inner surface of the holding section and the outer surface of the needle shield.

In another embodiment, the cap of the needle shield remover may be constructed from a thermoplastic biopolymer, such as, for example bio-polyethylene or bio-polypropylene. As an alternative, the cap may be made from a recycled polymer such as recycled polyethylene or recycled polypropylene. Using bio-polymers or recycled polymers may reduce the environmental impact or carbon footprint of the needle shield remover. As an alternative, the cap may be may be made from a biodegradable polymer. As another alternative, the cap may be made from a fossil based polymer.

In another embodiment, the elastic sleeve may be made from a thermoplastic elastomer with a medium to hard hardness value expressed in the shore Hardness. The thermoplastic elastomer may have a shore-A hardness ranging between 60 and 100, preferably between 65 and 95. The shore-A value may be measured after DIN EN 868 or after DIN ISO 48-4. The maximum shore A value or the value at 15 seconds may be used. Alternatively, the shore D hardness value is used. The thermoplastic elastomer may have a shore D value ranging between 10 and 60, preferably between 20 and 50. The shore D value may be measured according to the DIN EN 868 standard.

The thermoplastic elastomer may be based on styrene based tri-block copolymers (TPS), polyamide-polyether/polyester block copolymers (TPA), polyester-polyether block copolymers (TPC), thermoplastic polyolefin based copolymers (TPO) or polyurethane based copolymers (TPU) or a thermoplastic vulcanisate (TPV) consisting, for example of fully cured EPDM rubber particles encapsulated in a polypropylene (PP) matrix.

In another embodiment, the elastic sleeve is made from a rubber material such as a synthetic rubber or a natural rubber. The synthetic rubber may be a silicone elastomer or an ethylenepropylene-diene (EPDM) rubber. The silicone elastomer may be, for example, a polydimethylsiloxane elastomer (PDMS).

The materials selected for the elastic sleeve and/or the cap may fulfill the biocompatibility requirements according to the USP Class VI or ISO-10993 standards.

In an embodiment, the elastic sleeve or the flange of the elastic sleeve is made from a blend of the thermoplastic polymer used for the cap, for example a thermoplastic biopolymer, and the thermoplastic elastomer used for the elastic sleeve with a shore A hardness ranging between 65 and 90. The weight ratio between the thermoplastic elastomer and the (non -elastomeric) thermoplastic polymer ranges between 4:1 and 0.5:1. The adhesion in the overlap area between the flange of the elastic sleeve and the end wall may be increased by using the blend for the elastic sleeve thereby increasing the strength of the connection.

The present disclosure involves a method for applying a needle shield remover to a medicament delivery device including the following steps:
a) inserting a needle shield remover described above in a holding tool,
b) holding the distal end wall of the cap in the holding tool,
c) inserting the expansion tool into the passage of the distal end wall,
d) advancing the expansion tool in the proximal direction towards the holding section,
e) elastically deforming the holding section of the elastic sleeve using the expansion tool to a dimension greater than the outer dimension of the needle shield,
f) providing a medicament delivery device with an attached needle to the distal end covered by a needle shield,
g) advancing the medicament delivery device in the distal direction thereby inserting the needle shield in the elastically deformed or expanded holding section of the elastic sleeve,
h) retracting the expansion tool in the distal direction such that the holding section elastically relaxes to envelope and hold the needle shield.

Finally, the distal end wall of the cap is released from the holding tool and the medicament delivery device including the needle shield remover may be removed from the holding tool.

In an embodiment of the method, the expansion tool may be a tube with a fixed inner diameter greater than the outer dimension of the needle shield and a fixed outer diameter smaller than the diameter of the passage and further comprising the following step between steps c) and d):
i) applying pressurized gas to the interface between the outside surface of the tube and the inside surface of the elastic sleeve thereby creating an air cushion.

The air cushion may reduce the damage during expansion of the holding section and may allow for high-speed, high-volume manufacturing processes.

In another embodiment of the method, the expansion tool comprises multiple segments that are configured to radially expand during step e). The expansion tool thus has not a fixed outer dimension but may be expanded from a non-expanded to an expanded state for expanding the holding section of the elastic sleeve. In another embodiment, the expansion tool is a balloon that may be positioned between the expansion tool and the holding section and subsequently the balloon may be expanded by filling the balloon with pressurized gas.

The needle shield may be a rigid needle shield having a stiffness greater than the stiffness of the holding section of the elastic sleeve. As an alternative, the needle shield is a soft or flexible needle shield having a stiffness smaller than the stiffness of the stiffness of the holding section.

In another embodiment of the method, the pressurized gas is pressurized air or pressurized nitrogen that is cleaned before applying step i) and/or wherein the surface of the expansion tool is coated with a friction reducing layer.

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.

### LEGENDS TO THE FIGURES

- Figure 1:: Needle shield remover; longitudinal section,
- Figure 2:: Needle shield remover; outside surface cap,
- Figure 3:: Needle shield remover; 3D view longitudinal section,
- Figure 4:: Needle shield remover inserted in holding tool,
- Figure 5:: Needle shield remover, expansion tool and support pin advanced and elastic sleeve expanded,
- Figure 6:: Needle shield remover, expansion tool advanced and support pin retracted,
- Figure 7a:: 3D View of longitudinal section of the needle shield remover with expanded elastic sleeve aligned with prefilled syringe closed by a rigid needle shield (RNS),
- Figure 7b:: Axial alignment of the prefilled syringe with the needle shield remover,
- Figure 8:: Prefilled syringe in advanced position with RNS inserted into expanded elastic sleeve of the needle shield remover2.
- Figure 9:: Expansion tube of the holding tool in retracted position, RNS engaged with the elastic sleeve
- Figure 10:: Front section of a housing holding the prefilled syringe, the housing being engaged with the proximal section of the cap of the needle shield remover.
- Figure 11:: 3D Longitudinal section for the assembly of the needle shield remover with the housing of a medicament delivery device containing a prefilled syringe.

### DETAILED DESCRIPTION OF THE FIGURES

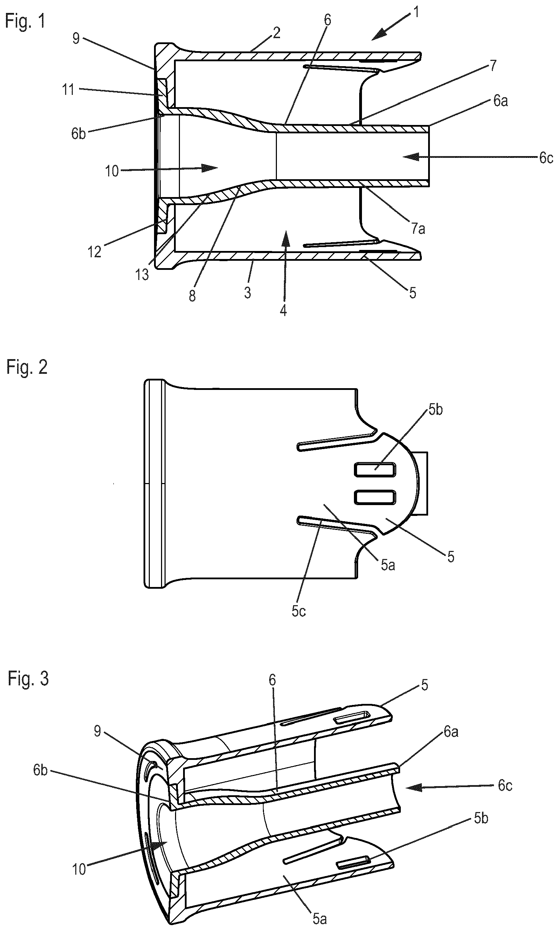

Figure 1 presents a longitudinal section of a needle shield remover 1 according to the present disclosure. The needle shield remover 1 includes a cap 2 with a side wall 3 closed by an end wall 9 present at the distal end of the cap 2. The proximal section 5 of the cap 2 includes slits 5c to provide a free standing elastic arm 5a, see Figure 2. The elastic arm 5 includes apertures 5b configured to engage complementary protrusions at the distal end of a housing of a medicament delivery device. The end wall 9 of the cap 2 extends radially inward from the side wall 3. The side wall 3 and the end wall 9 provide a bore 4 and an elastic sleeve 6 is coaxially arranged within the bore 4. The elastic sleeve 6 is provided as a cylindrical tube constructed from an elastic material. A flange 11 extends radially outward from the distal end 6b of the elastic sleeve. The end wall 9 of the cap 2 and the flange 11 of the elastic sleeve 6 mutually engage each other in an overlap area 12. The contacting surfaces of the flange 11 and the end wall 9 in the overlap area 12 may be structured, for example rippled or roughened, to increase the contact surface area and/or the mechanical interlock between the flange 11 and the end wall 9. The elastic sleeve 6 includes an opening 6c located at the proximal end 6a of the elastic sleeve 6. The elastic sleeve 6 includes a sleeve shaped holding section 7 and an entrance section 13 located between the holding section 7 and the flange 11. The inner surface7a of the holding section 7 may be textured or roughened. An opening or passage 10 is provided in the distal end wall 9 and the flange 11 connecting the inner volume or space of the elastic sleeve 6 to the outside. The passage 10 has an inner dimension configured to receive an expansion tool for radially expanding the elastic sleeve 6 and the holding section 7, see Figure 3. The overlap area may surround the passage 10.

The entrance section 13 has a has a variable dimension along the longitudinal axis of the cap 2, starting with an inner dimension equal to the inner dimension of the passage 10 and ending with an inner dimension equal to the inner dimension of the holding section 7. The entrance section 13 may be conically shaped or have the shape of a trumpet.

The elastic sleeve 7 includes an elastic wall 8. The wall 8 may be constructed as a braided structure or may have openings in the wall. In a preferred embodiment, the wall is made as a continuous structure or as a monolithic wall constructed from a thermoplastic elastomer. The thickness of the wall 8 may be a constant wall thickness or may vary between the flange 11, the entrance section 13 and the holding section 7. In a preferred embodiment, the wall thickness of the entrance section 13 is greater than the wall thickness of the holding section 7 and the wall thickness of the flange 11.

The needle shield remover presented in Figure 1, Figure 2 and Figure 3 presents the non-expanded state of the elastic sleeve 6 and/or holding section 7 of the elastic sleeve of the needle shield remover 1. After expansion of the elastic sleeve to an expanded state, a needle shield at the end of a container may be inserted into the expanded holding section 7 and subsequently the holding section may relax to envelope and hold the needle shield of the container thereby connecting the needle shield remover 1 to the needle shield. Removing the needle shield remover 1 by pulling the cap 2 will also remove the needle shield from the container. The container may be a prefilled syringe with a stacked needle.

An expansion tool fits into the passage 10 and the expansion tool may be moved into the proximal direction for expanding the entrance section 13 and/or the holding section 7 of the elastic sleeve 6. The wall thickness 8 for the entrance section may be adapted to accommodate for increased radial and/or axial forces applied onto the entrance section 13 during insertion of the expansion tool.

The cap 2 is preferably made from a thermoplastic homopolymer such as polyethylene or polypropylene. The cap may be made from a transparent homopolymer such as polycarbonate, polystyrene or polymethylmethacrylate. The cap may be made from a copolymer such as acrylonitrile-butadiene-styrene copolymer (ABS) or from a blend comprising polycarbonate and ABS. The thermoplastic homopolymer, the thermoplastic copolymer or the blend may be based on fossil based raw materials or may be based on raw materials from natural resources for obtaining biopolymers. Examples of biopolymers may be bio-polyethylene or bio-polypropylene.

The elastic sleeve may be made form a thermoplastic elastomer such as a styrene based tri-block copolymers (TPS), a polyamide-polyether/polyester block copolymers (TPA), polyester-polyether block copolymers (TPC), thermoplastic polyolefin based copolymers (TPO) or polyurethane based copolymers (TPU). The thermoplastic elastomer used for the sleeve has an elasticity or young's modulus that is below the elasticity or young's modulus of the cap material. Examples of the thermoplastic elastomers may be medical grade materials. For example a Santoprene^{™} thermoplastic vulcanizate (TPV, Celanese company) with a shore-A hardness of 94 ( measured after DIN EN 868), or a thermoplastic polyester elastomer Hytrel ^{®} with a shore-D value of 26 (TPE, Dupont) may be used. Alternatively, blends comprising a thermoplastic elastomer and a polyolefin (polyethylene or polypropylene) may be used.

The elastic sleeve 6 or the flange 11 of the elastic sleeve may be made from a blend of the thermoplastic elastomer and the thermoplastic polymer used for the cap. Adding the polymer used for the cap to the material used for the elastic sleeve may be beneficial to increase the compatibility between the two polymeric materials used for the cap and the elastic sleeve and therewith adhesion at the interface between the flange 11 and the end wall 9 in the overlap area 12.

The materials selected for the elastic sleeve and/or the cap may fulfill the biocompatibility requirements according to the USP Class VI and/or ISO-10993 standards.

Thermoplastic polymers are selected for the cap and for the elastic sleeve as they allow for injection molding of the cap and of the elastic sleeve. Preferably, the needle shield remover is produced using two-component injection molding whereby the two thermoplastic polymers are subsequently molded in a single mold.

The inner dimension of the passage 10 is greater than the outer dimension of the needle shield such that there is a clearance between the needle shield and the passage that is available for the expansion tool. The expansion tool has an outer dimension that fits into the passage and an inner dimension greater than, or expandable to a dimension greater than, the outer dimension of the needle shield. The holding section 7 of the elastic sleeve has an inner dimension in the relaxed, non-expanded state as presented in Figures 1 to 3, smaller than the outer dimension of the needle shield.

The method for applying the needle shield remover to a needle shield or to a syringe closed by a needle shield or to a medicament delivery device encasing a syringe closed the needle shield is presented in the following. The method may include steps for attaching the needle shield remover to the medicament delivery device or for attaching the needle shield remover to the housing of a medicament delivery device. The needle shield remover may be engaged first with the needle shield followed by attachment to the housing or vice versa, first the needle shield remover is attached to the housing followed by engaging the needle shield.

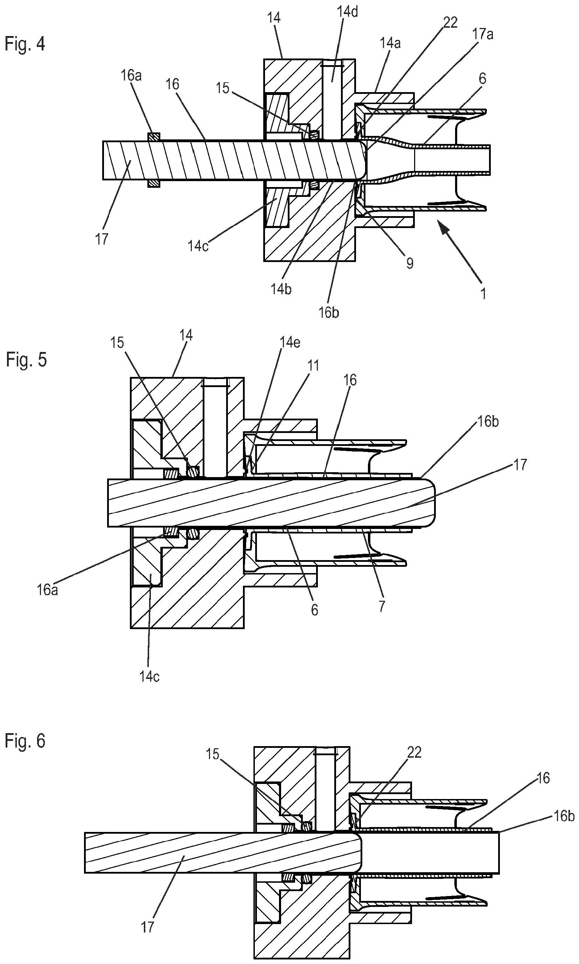

In a first step, the needle shield remover 1 is inserted into a holding tool 14 as depicted in Figure 4. The holding tool includes a holding section 14a engaging the distal end wall 9 of the needle shield remover 1. For example a vacuum may be applied via an opening 14d in the holding tool 14. Alternatively, the needle shield remover 1 may be fixated to the holding tool using releasable clamping features. The holding tool 14 may include a passage 14b for engaging and axially guiding an expansion tube 16. The holding tool 14 may be closed by an insert 14c compressing a sealing element or seal 15, for example an O-ring, towards a sealing seat in the holding tool 14. The seal 15 surrounds and seals the outer surface of the expansion tube 16 such that a vacuum may be applied via the opening 14d, or alternative pressurized gas may be applied via the opening 14d. The seal 15 provides for a sliding seal in that the expansion tube 16 may be moved back and forth in the passage 14b of the holding tool 14. The seal may be lubricated. A second seal 22 may be formed between the holding tool 14 and the distal end wall 9 of the needle shield remover (see Figure 4). A circumferential rim 14e may protrude from the surface of the holding tool 14 pointing towards the needle shield remover. The rim 14e elastically deforms the distal end wall of the flange 11 when the holding tool holds the needle shield remover. The rim 14e with the locally deformed flange 11 provides for the second sealing 22 (see Figures- 5 and 6). The second sealing 22 and the seal 15 allow for applying an under - or overpressure to the opening 14d

The expansion tube 16 is a cylindrical, preferably thin walled tube with a distal end 16b facing towards the needle shield remover 1 and a flange 16a at the proximal end that may be used for holding and axially shifting the expansion tube 16. The expansion tube may surround a support or guide pin 17 for mechanically supporting and guiding the expansion tube 16 through the passage 14b and through insert 14c of the holding tool 14. The guide pin 17 may have a dome shaped distal end 17a to facilitate expansion of the elastic sleeve 6 or to prevent damage to the elastic sleeve during high speed manufacturing processes.

In a next step, the expansion tube 16 and support pin 17 have been advanced towards the needle shield remover, as depicted in Figure 5. The elastic sleeve 6 and the holding section 7 are radially expanded by the expansion tube.

In a subsequent step, the support pin 17 has been retracted thus leaving the inner volume at the distal end of the expansion tube 16 available for needle shield insertion, see Figure 6.

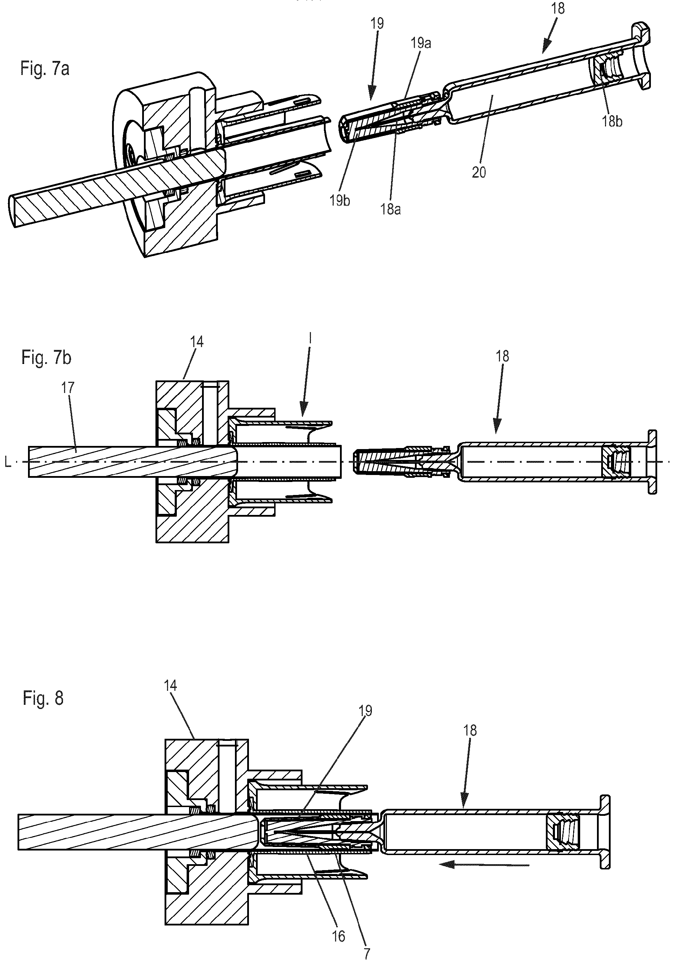

A prefilled syringe 18 may be axially aligned with the axis of the expansion tube 16 as shown in Figures 7a and 7b. The prefilled syringe 18 includes a glass wall closed by a stopper 18b at the proximal end, and including a hollow needle 18a at the distal end. A liquid medicament 20 may be present within the prefilled syringe 18 and advancing the stopper 18b by a drive mechanism expels medicament from the prefilled syringe via the hollow needle 18a. The prefilled syringe 18 is closed by a needle shield which may be a flexible (or soft) needle shield or a rigid needle shield 19 (RNS). The RNS includes an outer wall 19a and an elastic insert 19b surrounding the needle 18a. The RNS fits onto the distal end of the prefilled syringe via a press fit thereby providing a barrier between the outside and the needle 18a preventing the needle from contamination. The prefilled syringe will be aligned with the longitudinal axis L of the expansion tube 16 (Figure 7b) and in a subsequent step the prefilled syringe 18 may be advanced towards the holding tool or, alternatively, the holding tool 14 is advanced towards the prefilled syringe 18. The needle shield 19 is inserted into the opening of the expansion tube 16, as presented in Figure 8.

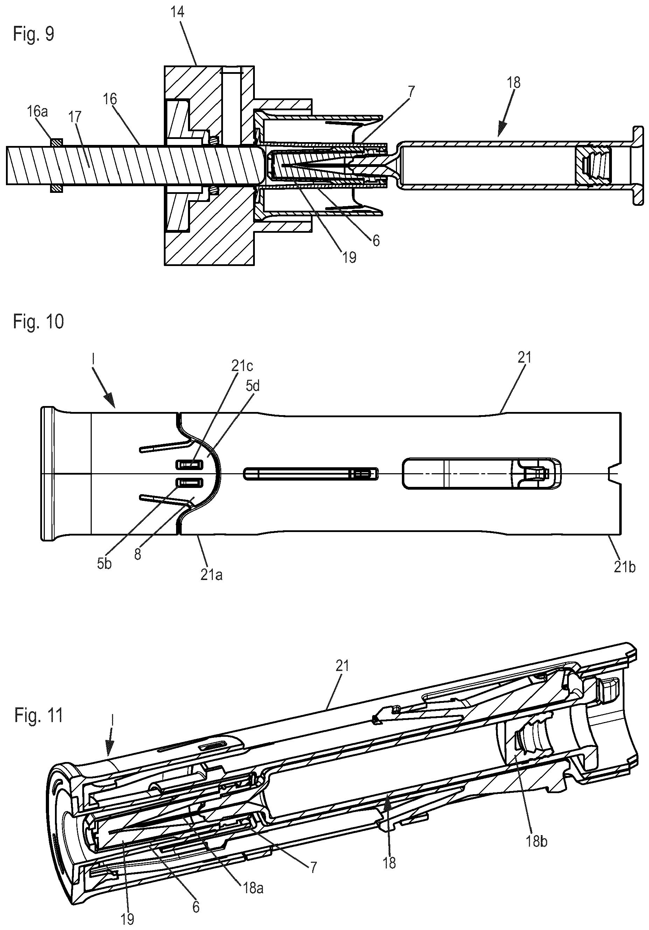

In a subsequent step presented in Figure 9, the expansion tube is retracted such that the elastic sleeve 6 and the holding section 7 radially relax towards the non-expanded state until the holding section 7 abuts and holds the rigid needle shield 19 in a friction fit and/or form fit engagement. The rigid needle shield is now coupled to the needle shield remover 1.

The prefilled syringe 18 may be part of a medicament delivery device such as an auto injector. The auto injector may have a drive mechanism for advancing the stopper 18b in the prefilled syringe for expelling medicament from the medicament delivery device. The drive mechanism may be spring driven and include a piston rod for advancing the stopper 18b in the prefilled syringe. The medicament delivery device may include a housing 21 as presented in Figure 10. The housing may enclose the drive mechanism and/or the prefilled syringe 18. The housing may enclose and provide mechanical support to a syringe holder for holding the prefilled syringe 18. The housing has a proximal end 21b that may be attachable to another housing part of a re-usable medicament delivery device. The housing 21 may be a disposable housing attachable to a reusable drive mechanism. Alternatively, the housing 21 encloses a disposable medicament delivery device enclosing both the prefilled syringe and the drive mechanism.

The distal end 21a of the housing 21 is attachable to the cap 2 of the needle shield remover 1 during or after insertion of the needle shield into the opening provided by the expansion tube (see Figures 7a and 8). The proximal end 5d of the elastic arm 5a may have a curved shape that fits into a complementary shape at the distal end 21a of the housing to ensure correct alignment of the housing 21 and the needle shield remover. Protrusions 21c protrude radially outward from the housing 21 or from a housing part connected to the housing 21 and the protrusions 21c fit into the apertures 5b of the cap. The needle shield remover 1 may snap fit onto the housing 21 of the medicament delivery device. Preferably, the connection between the needle shield remover and the housing may be established before retracting the expansion tube 16. A longitudinal section of the needle shield remover 1 attached to the housing of the medicament delivery device is presented in Figure 11. The elastic sleeve 6 tightly fits around the RNS 19 and by pulling the cap 2 of the needle shield remover 1, also the RNS will be removed such that the needle 18a is available for injection of the medicament from the prefilled syringe 18.

### PART ANNOTATION

| | | | |
|---|---|---|---|
| 1 | Needle shield remover | 14c | Insert |
| 2 | Cap | 14d | Opening |
| 3 | Side wall | 14e | Protruding rim |
| 4 | Bore | 15 | Seal |
| 5 | Proximal section | 16 | Expansion tube |
| 5a | Elastic arm | 16a | Flange |
| 5b | Aperture | 16b | Distal end |
| 5c | Slit | 16c | Proximal end |
| 5d | Proximal end | 17 | Support pin |
| 6 | Elastic sleeve | 17a | Distal end |
| 6a | Proximal end | 18 | Prefilled syringe |
| 6b | Distal end | 18a | Needle |
| 6c | Opening | 18b | Stopper |
| 7 | Holding section | 19 | Rigid needle shield |
| 7a | Inner surface | 19a | Outer wall |
| 8 | Wall | 19b | Elastic insert |
| 9 | Distal end wall | 20 | Liquid medicament |
| 10 | Passage | 21 | Housing |
| 11 | Flange | 21a | Distal end |
| 12 | Overlap area | 21b | Proximal end |
| 13 | Entrance section | 21c | Protrusion |
| 14 | Holding tool | 22 | Second seal |
| 14a | Holding section | L | Longitudinal axis |
| 14b | Passage | | |

## Claims

1. A needle shield remover (1) for a medicament delivery device comprising a syringe with an attached needle to the distal end covered by a needle shield (19), the needle shield remover comprising:
- a cap (2) comprising a side wall (3) providing a bore (4), the proximal section (5) of the side wall (3) is configured for attachment to the medicament delivery device,
- an elastic sleeve (6) positioned in the bore (4), wherein the elastic sleeve (6) comprises a holding section (7) for holding the needle shield provided with an inner diameter smaller than the outer diameter of the needle shield (19), the elastic sleeve (7) comprising a wall (8) constructed from an elastic material,
- a distal end wall (9) connecting the elastic sleeve (6) to the cap (2),
**characterized by**
a passage (10) provided in the distal end wall (9) connecting the inner space of the elastic sleeve (6) to the exterior, the passage (10) being configured for receiving an expansion tool (16) for expanding the elastic sleeve, wherein, the inner dimension of the passage (10) is greater than the outer diameter of the needle shield (19).

2. The needle shield remover according to claim 1, manufactured by multi-component injection molding of at least two different thermoplastic polymers for the cap (2) and for the elastic sleeve (6) whereby an elastomer is selected for the elastic sleeve.

3. The needle shield remover according to claim 2, wherein the elastic sleeve (6) is a cylindrical sleeve comprising a flange (11) that extends radially outward from the distal end (6b) of the elastic sleeve (6).

4. The needle shield remover according to claim 3, wherein the side wall (3) of the cap is a cylinder and wherein the end wall (9) depends radially inward from the side wall.

5. The needle shield remover according to claim 3 or claim 4, wherein the flange (11) of the elastic sleeve (6) and the end wall (9) are connected to each other in an overlap area (12).

6. The needle shield remover according to any of claims 3 to 5, wherein the elastic sleeve (6) comprises an entrance section (13) configured for receiving the distal end of the expansion tool, the entrance section (13) positioned between the holding section (7) and the flange (11).

7. The needle shield remover according to any of claims 1 to 6, wherein the inner surface (7a) of the holding section (7) of the elastic sleeve (6) is roughened or textured.

8. The needle shield remover according to any of claims 1 to 7, wherein the cap (2) is made from a thermoplastic biopolymer.

9. The needle shield remover according to claim 8, wherein the elastic sleeve (6) is made from a thermoplastic elastomer with a shore-A hardness ranging between 60 and 100.

10. The needle shield remover according to claim 9 wherein the elastic sleeve (6) is made from a blend of the thermoplastic biopolymer used for the cap and the thermoplastic elastomer with a shore-A hardness ranging between 60 and 100.

11. A method for applying a needle shield remover to a medicament delivery device comprising the following steps:
a) inserting a needle shield remover (1) according to any of claims 1 to 10 in a holding tool (14),
b) holding the distal end wall (9) in the holding tool (14),
c) inserting the expansion tool (16) in the passage (10) of the distal end wall (9),
d) advancing the expansion tool (16) in the proximal direction towards the holding section (7),
e) elastically deforming the holding section (7) of the elastic sleeve (6) using the expansion tool (16) to a dimension greater than the outer dimension of the needle shield,
f) providing a medicament delivery device with an attached needle (18a) to the distal end covered by a needle shield (19),
g) inserting the needle shield (19) in the elastically deformed holding section (7) of the elastic sleeve (6),
h) retracting the expansion tool (16) in the distal direction such that the holding section (7) elastically relaxes to envelope and hold the needle shield (19).

12. The method according to claim 11 wherein the expansion tool is a tube with an inner diameter greater than the outer dimension of the needle shield and an outer diameter smaller than the diameter of the passage and further comprising the following step between steps c) and d):
i) applying pressurized gas to the interface between the outside surface of the tube and the inside surface of the elastic sleeve thereby creating an air cushion.

13. The method according to claim 11 or 12 wherein the expansion tool comprises multiple segments that are configured to radially expand during step e).

14. The method according to claim 13 wherein the needle shield is a rigid needle shield having a stiffness greater than the stiffness of the holding section of the elastic sleeve.

15. The method according to claim 14 wherein the pressurized gas is pressurized air which is cleaned before applying step i) and/or wherein the surface of the expansion tool is coated with a friction reducing layer.
